# EUROPEAN PATENT SPECIFICATION

(11) **EP 3 362 113 B1**
(45) Date of publication and mention of the grant of the patent: **04.08.2021**
(21) Application number: 16819677.2
(22) Date of filing: 15.12.2016
(51) Int. Cl.: A61M 1/00, G01L 9/00

(54) **OPTICAL PRESSURE MEASUREMENT FOR OPHTHALMIC SURGICAL FLUIDICS**
OPTISCHE DRUCKMESSUNG FÜR OPHTHALMISCHE CHIRURGISCHE FLUIDIK
MESURE DE LA PRESSION OPTIQUE POUR CHIRURGIE FLUIDIQUE OPHTALMIQUE

(30) Priority: 30.12.2015 US 201562272946 P
(43) Date of publication of application: 22.08.2018
(73) Proprietor: Alcon Inc., 1701 Fribourg (CH)
(72) Inventor: BAXTER, Vincent A., Lake Forest California 92630 (US); WILSON, Daniel J., Lake Forest California 92630 (US)
(74) Representative: Hanna Moore + Curley
(86) International application number: PCT/IB2016/057677
(87) International publication number: WO 2017/115199

(56) References cited:
- EP-A1- 1 612 532
- US-A1- 2004 261 534
- US-A1- 2015 164 690

## Description

### TECHNICAL FIELD

The present disclosure is directed to ophthalmic surgical systems and methods of measuring aspiration and/or irrigation fluid pressure.

### BACKGROUND

Fluid pressure within an eye of a patient, often referred to as "intraocular pressure," should be maintained at a relatively constant value during an ophthalmic surgical procedure to avoid adverse physiological effects. Ophthalmic surgical procedures frequently remove body tissue from the eye, as well as fluid and other anatomy, in a process referred to as aspiration. To counteract the decrease in fluid pressure resulting from aspiration, the surgical probe or another surgical device can deliver fluid into the eye in a process referred to as irrigation.

A fluidics module of a surgical console and a fluidics cassette that interfaces with the fluidics module can be utilized to maintain intraocular pressure through aspiration and irrigation. The fluidics cassette generally includes tubing involved in guiding aspiration fluid away from the eye and irrigation fluid towards the eye. The fluid pressures of the aspiration fluid and the irrigation fluid must be monitored in order to maintain appropriate intraocular pressure.

Some conventional systems determine fluid pressures based on a position of a portion of the fluidics cassette in the surgical console. In the systems, movement of the fluidics module or fluidics cassette can impact the pressure readings, introducing measurement inaccuracies. While manufacturers strive to minimize unwanted movement, the fluidics module and fluidics cassette are susceptible to such movement during the surgical procedure. For example, the geometry and force of a mechanism clamping the fluidics cassette and the fluidics module, the operation of rollers and valves of the fluidics module, and/or the pulling of tubing can result in unwanted movement. As a result of such movement, accurately determining the aspiration and irrigation fluid pressures can be difficult.

Reference is made to EP 1 612 532 which has been cited as representative of the state of the art.

### SUMMARY

It will be appreciated that the scope of the invention is in accordance with the claims. There is provided an ophthalmic surgical system in accordance with claim 1 and a method of determining a pressure within an ophthalmic surgical system in accordance with claim 13. Further features are set out in the dependent claims. Aspects, examples and embodiments of the disclosure which do not fall within the scope of the claims are provided for illustration purposes only.

According to one aspect, the present disclosure describes an ophthalmic surgical system including at least one light source. The at least one light source is configured to output light towards a fluidics cassette in a manner that a first portion of the light reflects from a deflectable diaphragm of the fluidics cassette, and in a manner that a second portion of the light reflects from a reference portion of the fluidics cassette. The diaphragm is configured to deflect relative to the reference portion in response to a pressure associated with a fluid within the fluidics cassette. The system also includes at least one sensor configured to receive the first portion of the light reflected from the diaphragm and the second portion of the light reflected from the reference portion. The system further includes a computing device in communication with the at least one sensor. The computing device is configured to determine the pressure associated with the fluid within the fluidics cassette based on the received first and second portions of the light.

Another aspect of the present disclosure is directed to an ophthalmic surgical system including at least one light source. The at least one light source is configured to output light towards a fluidics cassette in a manner that a first portion of the light reflects from a first region of diaphragm of the fluidics cassette, and in a manner that a second portion of the light reflects from a component of the fluidics cassette spaced from the first region of the diaphragm. The diaphragm is configured to be deflected in response to a pressure associated with a fluid within the fluidics cassette. The system also includes at least one sensor configured to receive the first portion of the light reflected from the first region of the diaphragm and the second portion of the light reflected from the mount for the diaphragm. The system further includes a computing device in communication with the at least one sensor. The computing device is configured to determine the pressure associated with the fluid within the fluidics cassette based on the received first and second portions of the light.

A third aspect of the disclosure is directed to a method of determining a pressure within an ophthalmic surgical system. The method includes controlling, using a computing device, at least one light source to output light towards a fluidics cassette such that a first portion of the light reflects from a region of a diaphragm of the fluidics cassette and such that a second portion of the light reflects from a component of the fluidics cassette spaced from the region of the diaphragm. The diaphragm is configured to be deflected in response to a pressure associated with a fluid within the fluidics cassette. The method also includes receiving at the computing device, from at least one sensor, a first signal representative of the first portion of the light reflected from the region of the diaphragm and a second signal representative of the second portion of the light reflected from the component of the fluidics cassette remote from the region of the diaphragm. The method further includes determining, using the computing device, the pressure associated with the fluid within the fluidics cassette based on the received first and second signals.

The various aspects of the disclosure may include one or more of the following features. The system may further include a beam splitter configured to direct the first portion of the light towards the diaphragm of the fluidics cassette and the second portion of the light towards the reference portion. The at least one sensor can include a first sensor configured to receive the first portion of the light reflected from the diaphragm; and a second sensor configured to receive the second portion of the light reflected from the reference portion. The at least one light source may include a first light source configured to output the first portion of the light; and a second light source configured to output the second portion of the light. The at least one sensor can include a first sensor configured to receive the first portion of the light reflected from the diaphragm; and a second sensor configured to receive the second portion of the light reflected from the reference portion. The at least one light source may be configured to output light towards a fluidics cassette such that a third portion of the light reflects from a second diaphragm of the fluidics cassette and a fourth portion of the light reflects from a second reference portion of the fluidics cassette. The further diaphragm is configured to be deflected in response to a pressure associated with a second fluid within the fluidics cassette. The at least one sensor is configured to receive the third portion of the light reflected from the second diaphragm and the fourth portion of the light reflected from the second reference portion. The computing device is configured to determine the pressure associated with the further fluid within the fluidics cassette based on the received third and fourth portions of the light. The pressure associated with the fluid within the fluidics cassette may be representative of an irrigation pressure. The pressure associated with the second fluid within the fluidics cassette can be representative of an aspiration pressure.

The at least one light source can include a laser source or a laser diode. The system can further include a surgical console housing the at least one light source, the at least one sensor, and the computing device. The system may further include the fluidics cassette. The computing device can be configured to determine the pressure associated with the fluid within the fluidics cassette by: determining a first distance between the at least one sensor and the diaphragm based on the received first portion of the light reflected from the diaphragm; and determining a second distance between the at least one sensor and the reference portion based on the received second portion of the light reflected from the reference portion. The computing device may be configured to determine the pressure associated with the fluid within the fluidics cassette by: calculating a displacement of the diaphragm by subtracting the second distance from the first distance. The computing device can configured to determine the pressure associated with the fluid within the fluidics cassette by: correlating the displacement of the diaphragm to the pressure associated with the fluid within the fluidics cassette.

The component of the fluidics cassette spaced from the first region of the diaphragm can include at least one of: a second region of the diaphragm; and a mount for the diaphragm. The first region of the diaphragm may include a central region of the diaphragm. The second region of the diaphragm may include a peripheral region of the diaphragm.

The various aspects of the disclosure may also include one or more of the following features. Determining the pressure associated with the fluid within the fluidics cassette can include determining a first distance between the at least one sensor and the region of the diaphragm based on the received first signal; and determining a second distance between the at least one sensor and the component of the fluidics cassette spaced from the region of the diaphragm based on the received second signal. Determining the pressure associated with the fluid within the fluidics cassette may include: calculating a displacement of the diaphragm by subtracting the second distance from the first distance. Determining the pressure associated with the fluid within the fluidics cassette can include: correlating the displacement of the diaphragm to the pressure associated with the fluid within the fluidics cassette.

It is to be understood that both the foregoing general description and the following drawings and detailed description are exemplary and explanatory in nature and are intended to provide an understanding of the present disclosure without limiting the scope of the present disclosure. In that regard, additional aspects, features, and advantages of the present disclosure will be apparent to one skilled in the art from the following.

### BRIEF DESCRIPTION OF THE DRAWINGS

The accompanying drawings illustrate embodiments of the systems, devices, and methods disclosed herein and together with the description, serve to explain the principles of the present disclosure.
FIG. 1 is an illustration of an example ophthalmic surgical system.
FIG. 2 is a block diagram of an ophthalmic surgical system.
FIG. 3 is an illustration showing a perspective view of a fluidics module of a surgical console.
FIG. 4A is an illustration showing a front view of a fluidics cassette.
FIG. 4B is an illustration showing a perspective view of the fluidics cassette of FIG. 4A.
FIGs. 5A, 5B, and 5C are illustrations showing measurement of pressure within a fluidics cassette using an optical pressure sensor of a fluidics module.
FIG. 6 is a flow diagram of an example ophthalmic surgical method.

These figures will be better understood by reference to the following Detailed Description.

### DETAILED DESCRIPTION

For the purposes of promoting an understanding of the principles of the present disclosure, reference will now be made to the implementations illustrated in the drawings and specific language will be used to describe them. It will nevertheless be understood that no limitation of the scope of the disclosure is intended. Any alterations and further modifications to the described devices, instruments, methods, and any further application of the principles of the present disclosure are fully contemplated as would normally occur to one skilled in the art to which the disclosure relates. In particular, it is fully contemplated that the features, components, and/or steps described with reference to one or more implementations may be combined with the features, components, and/or steps described with reference to other implementations of the present disclosure. For simplicity, in some instances the same reference numbers are used throughout the drawings to refer to the same or like parts.

The present disclosure relates generally to devices, systems, and methods for determining the irrigation pressure and the aspiration pressure within a fluidics cassette. In the implementations described herein, the irrigation pressure and the aspiration pressure may be indicative of intraocular pressure within a patient's eye. In some implementations, the fluidics cassette may include an irrigation diaphragm and an aspiration diaphragm. These diaphragms may be respectively configured to deflect in response to the pressure (e.g., fluid pressure and/or vacuum pressure) associated with irrigation fluid and aspiration fluid within the cassette. By measuring the deflection with a fluidics module of a surgical console, a user/controller may determine the pressure. In some implementations, the fluidics module includes an optical pressure sensor that measures the deflection of the irrigation diaphragm and the aspiration diaphragm. In some implementations, the optical pressure sensor may include one or more laser sources that output light that is reflected by the irrigation diaphragm and/or the aspiration diaphragm. The reflected light can be received at one or more sensors, and a computing device can determine the deflection based on the reflected light received at the sensor. The distance can vary as the diaphragms are displaced.

In some implementations as a point of reference, the optical pressure sensor may also measure the distance to a relatively more stationary part of the cassette, such as a mount for the diaphragm. In that regard, the diaphragm is configured to deflect relative to the reference location in response to pressure within the cassette. For example, measuring the distance to the mount can account for any unwanted movement of the fluidics module and/or the cassette. By subtracting the distance to the mount from the distance to the diaphragm, the displacement of the diaphragm resulting from pressure within the cassette can be determined. The computing device can determine the pressure associated with irrigation fluid and/or aspiration fluid based on the displacement of the diaphragm.

The devices, systems, and methods of the present disclosure provide numerous advantages over conventional systems. In particular, the pressure associated with the irrigation fluid and/or aspiration fluid may be more accurately determined by using the reference measurement location on the fluidics cassette. Displacement of the diaphragm resulting from pressure can be separated from unwanted movement of the fluidics cassette and/or the fluidics module. Accordingly, the geometry and force of the mechanism clamping the fluidics cassette and the fluidics module, the operation of rollers and valves of the fluidics module, the pulling of tubing, and/or other sources of unwanted movement can be accounted for. The devices, systems, and methods disclosed herein may also permit easier manufacturing than prior systems. For example, in order to prevent unwanted movement, cassettes were rigidly coupled to the fluidics module of the surgical console, with high clamp tension and force, in some systems. The disclosure herein allows for the clamp tension and force to be reduced because the pressure measurement accounts for any movement of the cassette and/or the fluidics module.

Additionally, calibration of the fluidics cassette can be more efficiently completed based on the present disclosure. In particular, zero cassette calibration is typically performed just before the surgical procedure when the cassette is inserted into the fluidics module of the surgical console. According to the present disclosure, this calibration step can be removed from the surgical setup workflow, which allows for a more efficient set up for the user, such as the surgeon or other medical profession. Zero cassette calibration is used to determine the diaphragm position when there is no pressure in the fluidics cassette. The zero cassette calibration may be completed during cassette manufacturing. The calibration data may be stored on a memory device associated with the cassette, such as a barcode that is read when the cassette is inserted into the fluidics module of the console. The calibration will not change when the cassette is inserted into the fluidics module, and the known zero cassette calibration data obtained during manufacturing can be advantageously utilized.

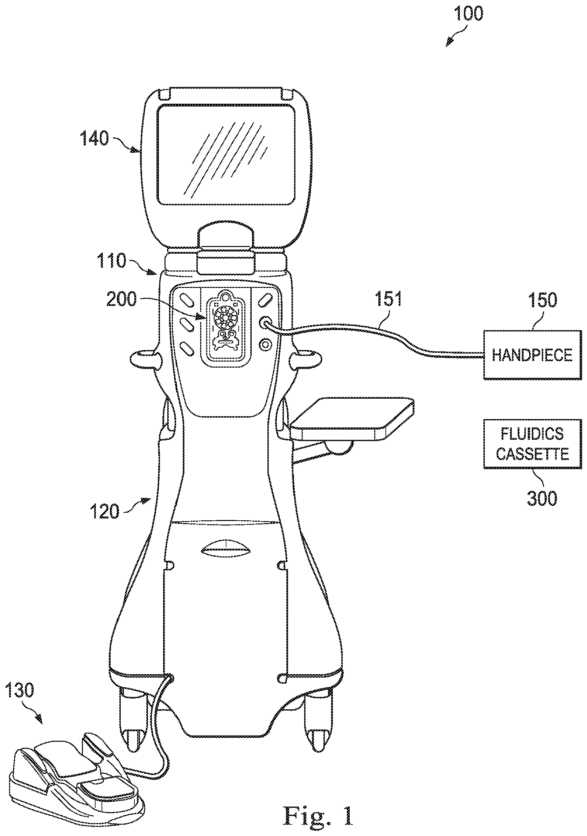

FIG. 1 illustrates an example ophthalmic surgical system 100. The system includes a surgical console 110 having a fluidics module 200, a fluidics cassette 300 configured to interface with the fluidics module 200, and a handpiece 150 that is configured to treat the patient's eye. The system 100 may be used to perform various ophthalmic surgical procedures including an anterior segment procedure, a posterior segment procedure, a vitreoretinal procedure, a vitrectomy procedure, a cataract procedure, and/or other desired procedures. The surgical console 110 includes a mobile base housing 120 and an associated display screen 140 showing data relating to system operation and performance during the procedure. The system 100 can also include a surgical footswitch 130 for controlling operation of the handpiece 150 and/or other system components during the procedure. The surgical footswitch 130 can be in wired or wireless communication with the surgical console 110.

One or more surgical devices, including the handpiece 150, can be communicatively coupled to the console 110. For example, the handpiece 150 may be in fluid and/or electrical communication with the console 110. One or more conduits 151, such as tubing configured to carry aspiration fluid and/or irrigation fluid, can extend between the console 110 and the handpiece 150. A distal portion of handpiece 150 may be inserted into the eye to treat an optical condition. In various embodiments, the handpiece 150 can be a cutting probe, a vitrectomy probe, a phacoemulsification probe, a laser probe, an ablation probe, a vacuum probe, a flushing probe, scissors, forceps, an infusion device, an irrigation device, an aspiration device, other suitable surgical device, and/or combinations thereof. For example, in a cataract procedure, a phacoemulsification probe can be used to reshape the lens of the patient's eye.

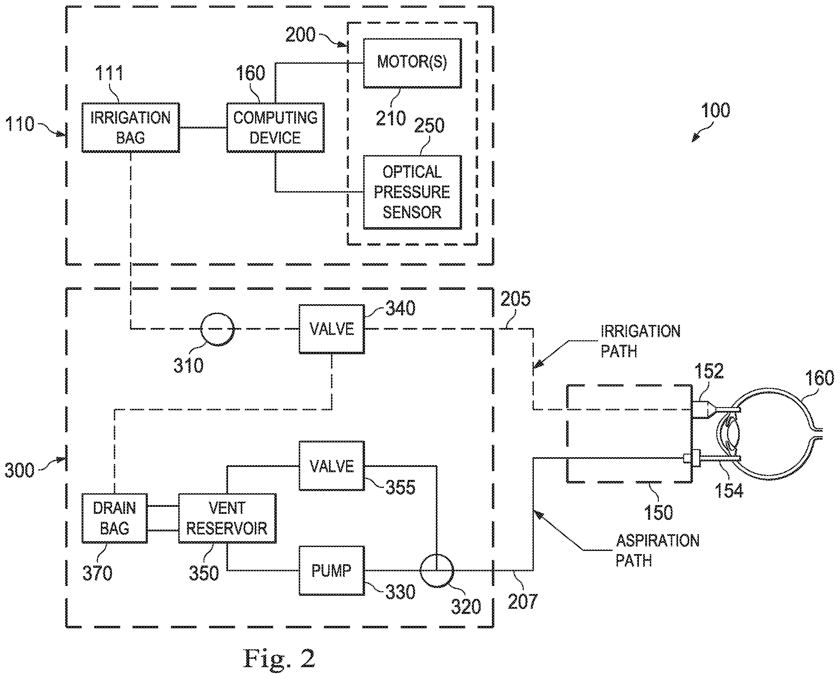

FIG. 2 illustrates additional features of an example handpiece 150. Here, the handpiece 150 is a phacoemulsification probe including a sleeve 152 and a tip 154. Irrigation fluid is delivered into an eye 160 using the sleeve 152. The tip 154 can vibrate at a high frequency to remove desired portions of the lens. Excised tissue, fluid within the eye, and/or other anatomy can be aspirated away the eye 160 through a lumen of the tip 154. While the sleeve 152 and the tip 154 are shown as spaced from one another to more clearly delineate an aspiration path 207 and an irrigation path 205 in FIG. 2, it is understood that the sleeve 152 can be circumferentially positioned around the tip 154.

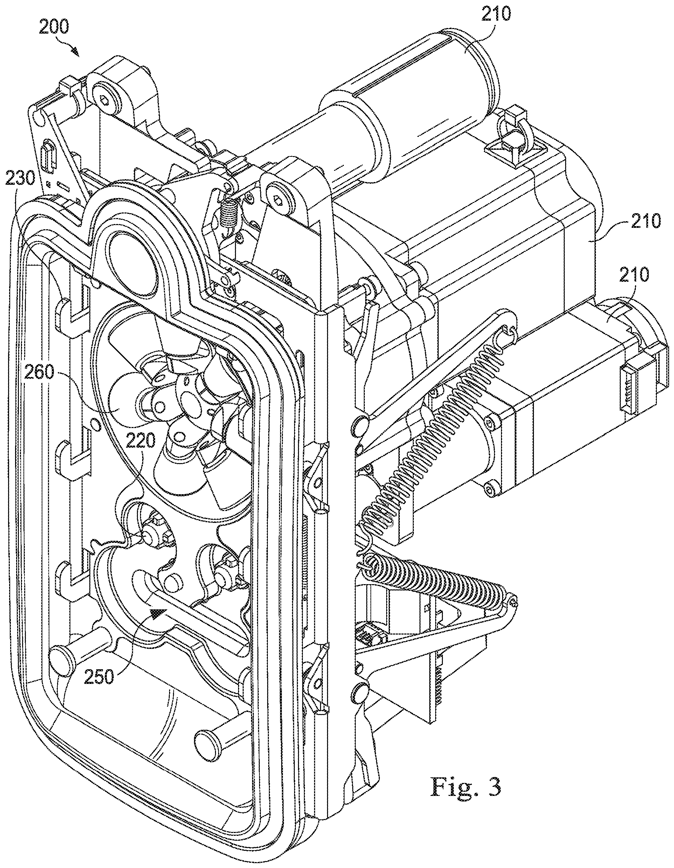

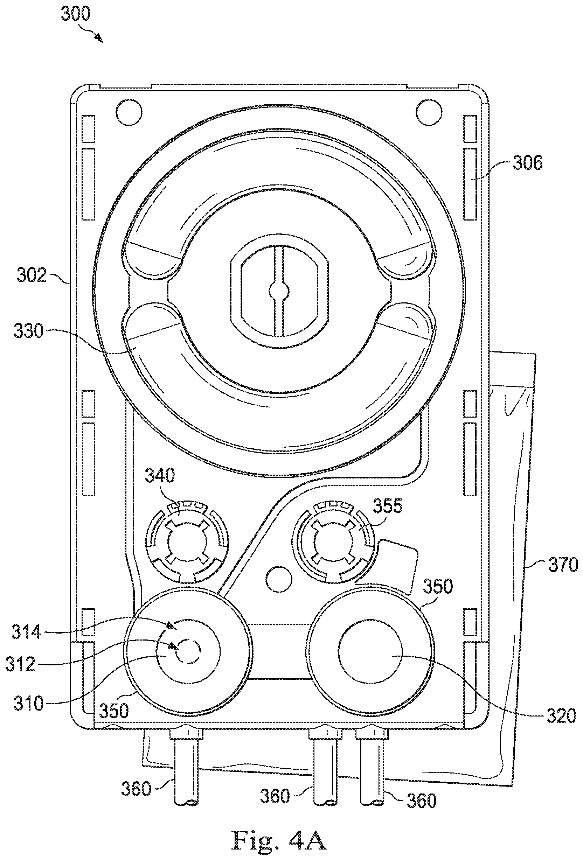

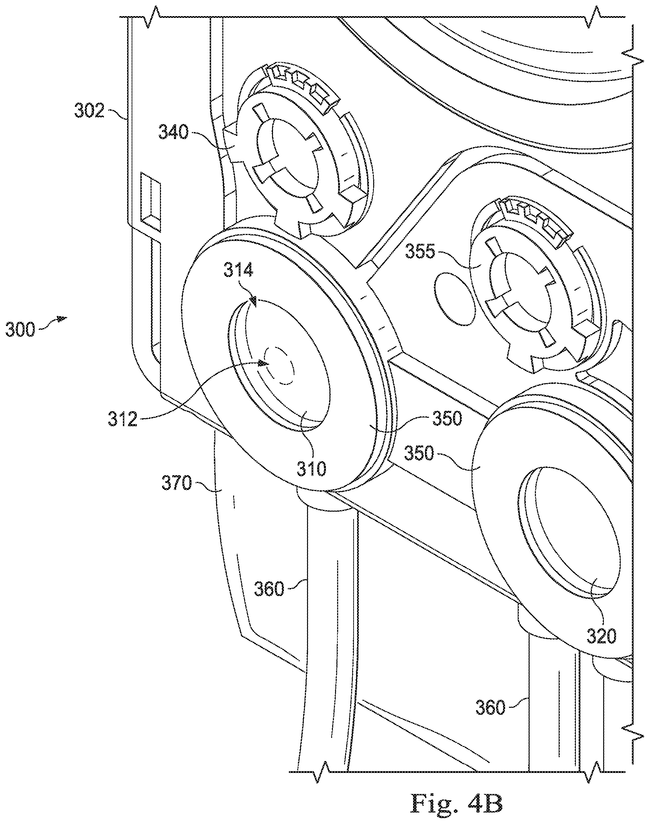

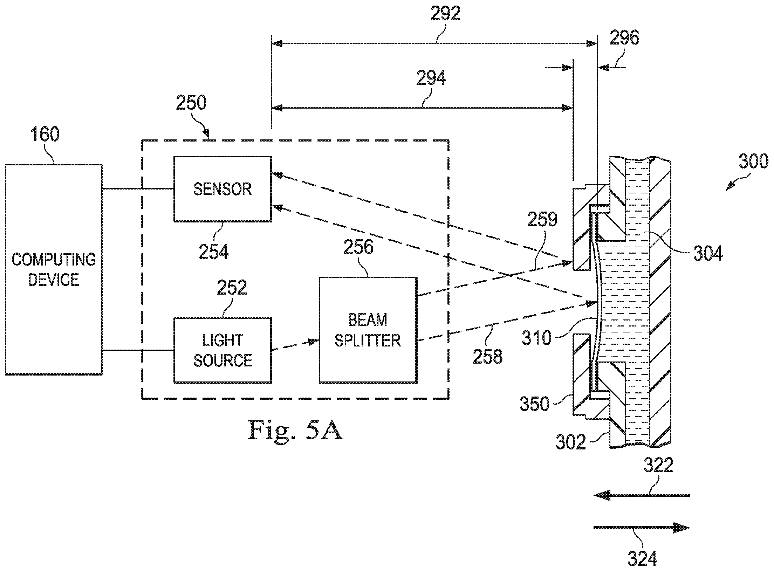

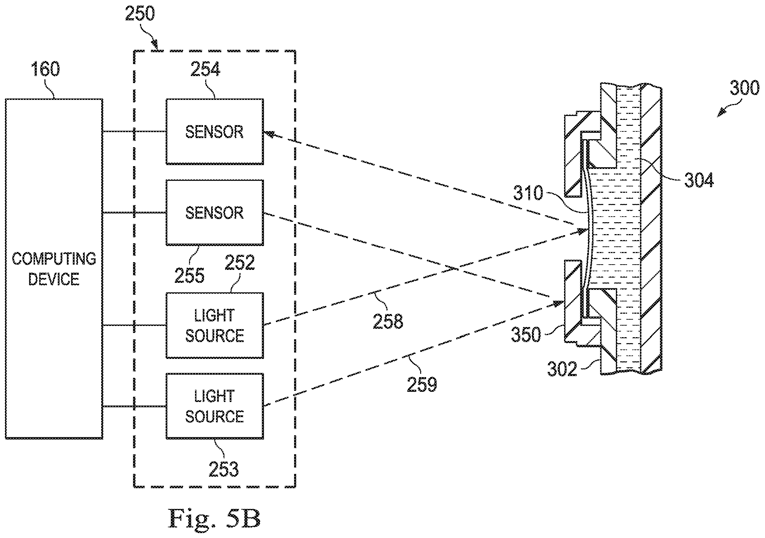

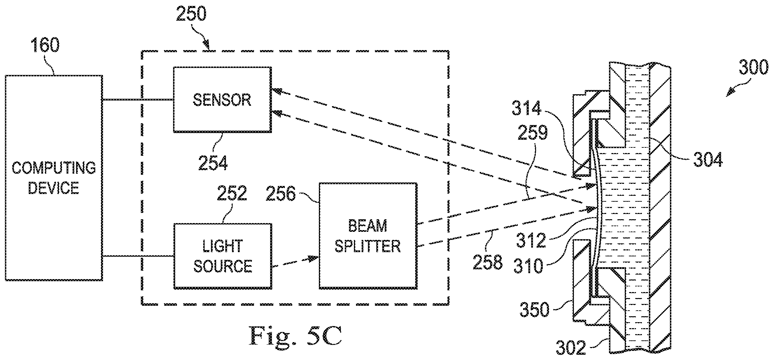

Embodiments of the fluidics module 200 of the surgical console 110 are illustrated in FIGs. 1, 2, and 3. The fluidics module 200 and the cassette 300 together facilitate delivery of irrigation fluid into the eye 160 along the irrigation path 205 and removal of aspiration fluid from the eye 160 along the aspiration path 207 during the surgical procedure. Embodiments of the cassette 300 are illustrated in FIGs. 4A and 4B, as well as FIGs. 5A, 5B, and 5C. The cassette 300 can be a consumable component that is removably coupled to the fluidics module 200. A different cassette 300 can be used for different surgical procedures. The cassette 300 can be discarded after the surgical procedure because it contacts biological material, whereas the fluidics module 200 (as well as the console 110) does not ordinarily contact biological material and is reused in different surgical procedures. The cassette 300 can be coupled to the fluidics module 200 such that the components of cassette 300 illustrated in FIG. 4A are adjacent to and interface with the components of the fluidics module 300 illustrated in foreground of FIG. 3.

Returning to FIG. 2, irrigation fluid can travel along the irrigation path 205 from the irrigation bag 110 to the sleeve 152 of the handpiece 150 and into the eye 160. A valve 340 within the irrigation path 205 can selectively control the flow of the irrigation fluid. Aspiration fluid can travel from the eye 160, through the tip 154 of the handpiece 150, along the aspiration path 207 to the drain bag 370. A pump 330, valve 355, and vent reservoir 350 can cooperate to selectively control the flow of the aspiration fluid. Rollers 260 (FIG. 3) of the fluidics module 200 contact and press against the pump 330 of the cassette 300 to urge aspiration fluid away from the eye 160 and towards the drain bag 370. Valve controls 220 (FIG. 3) of the fluidics module 200 engage and control the position of the valves 340, 355 of the cassette 300. Attachment members 230 of the fluidics module 200 releasably grasp grooves 306 (FIG. 4) of the cassette 300 to maintain the cassette 300 in position to interface with the fluidics module 200. Motors 210 of the fluidics module 200 can control operation of the rollers 210, the valve controls 220, and the attachment members 230. Tubes 360 (FIG. 4) extend between a body 302 of the cassette 330 and the drain bag 370 (FIG. 3) to deliver irrigation fluid and/or aspiration fluid.

Referring to FIGs. 4A and 4B, the fluidics cassette 300 includes an irrigation diaphragm 310 and an aspiration diaphragm 320. The diaphragms 310, 320 are flexible membranes that can be formed of any suitable material. In some exemplary embodiments, the material can include a metal, such as stainless steel or titanium, a plastic, a polymer, such as polytetrafluoroethylene (PTFE), other suitable materials, and/or combinations thereof. In that regard, the diaphragms 310, 320 are configured deflect, bend, and/or otherwise be displaced in response to the fluid pressure and/or the vacuum pressure within the cassette 300.

FIGs. 5A, 5B, and 5C show a cross-sectional view of a portion of the fluidics cassette 300. As illustrated, irrigation fluid 304 travels within the body 302 of the fluidics cassette 300. The irrigation fluid 304 flows adjacent to the diaphragm 310 such that the shape of the diaphragm 310 is influenced by the pressure associated with the irrigation fluid 304. The diaphragm 310 can deflect, such as in a bowed or arcuate manner, in the directions 322, 324 in response to the pressure associated with the irrigation fluid 304. For example, an increase in vacuum pressure along the irrigation path 205 can cause the diaphragm 310 to deflect inward, in the direction 324. A decrease in vacuum pressure along the irrigation path 205 can cause the diaphragm to deflect outward, in the direction 322. It understood that the aspiration diaphragm 320 can share many of the same features of the irrigation diaphragm 310. Thus, the aspiration diaphragm 320 can deflect in response to pressure associated with the aspiration fluid, as similarly described with respect to the irrigation diaphragm 310 and the irrigation fluid 304.

Referring again to FIGs. 4A and 4B, the diaphragms 310, 320 can include a central region 312 and a peripheral region 314. The central region 312 can be any suitably sized and shaped area in the center of the diaphragms 310, 320, such as the example circle shaped region illustrated in FIGs. 4A and 4B. The peripheral region 314 can be any suitably sized and shaped area of the diaphragms 310, 320 surrounding the central region 312, such as the example doughnut shaped region illustrated in FIGs. 4A and 4B. In some embodiments, the central region 312 can be displaced a relatively greater amount than the peripheral region 314 in response to the same pressure within the fluidics cassette 300. As shown in FIGs. 4A, 4B, 5A, 5B, and 5C, the diaphragms 310, 320 are surrounded by mounts 350. The mounts 350 can be rigidly affixed and/or integrally formed with the body 302 of the cassette 300. That is, the mounts 350 are stationary with respect to the body 302 of the cassette 300. Thus, displacement or deflection of the diaphragms 310, 320, relative to the mount 350, can be determined. In general, the diaphragms 310, 320 (e.g., the central region 312 or the peripheral region 314) deflects relative to the reference location (e.g., the mount 350 or the peripheral region 314) on the fluidics cassette 300.

Referring to FIGs. 2 and 3, the fluidics module 200 includes an optical pressure sensor 250 configured to detect pressure differentials indicative of the fluid pressure and/or vacuum pressure associated with irrigation fluid and/or aspiration fluid within the cassette 300. In particular, the optical pressure sensor 250 can be configured to measure the distance between the optical pressure sensor 250 and components of the fluidics cassette, such as the diaphragms 310, 320, and the mounts 350. A computing device (e.g., a computing device 160 of the surgical console 110) can determine the pressure associated with the irrigation fluid and/or aspiration fluid based on the measured distances. Other features of the optical pressure sensor 250 are illustrated in FIGS. 5A, 5B, and 5C. For example, the optical pressure sensor 250 can include a light source 252 and a sensor 254. The light source 252 can be configured to output light towards the cassette 300, the diaphragms 310, 320, and/or the mounts 350. In that regard, the light source 242 can be source of coherent light, such as a laser source or a laser diode, and/or other suitable source. The sensor 254 can be any suitable sensor configured to detect reflected portions of the light transmitted by the light source 252. For example, the sensor 254 can be a charge coupled device (CCD) sensor, a complementary metal-oxide-semiconductor (CMOS) sensor, and/or other suitable sensor. The optical pressure sensor 250 can be positioned on the fluidics module 200 so as to be near or proximate to the diaphragms 310, 320 when the cassette 300 interfaces with the fluidics module 200. The sensor 254 can generate an electrical signal representative of the received light. The light source 242 and/or the sensor 254 can be in communication with the computing device 160. The computing device 160, the light source 242, and/or the sensor 254 can be housed or disposed within the console 110.

The computing device 160 can be any suitable computer having a processor and a memory forming a processing circuit. The processor may execute computer instructions, such as those stored on the memory, to control various components of the system 100 described herein. The memory, which is typically a semiconductor memory such as RAM, FRAM, or flash memory, interfaces with the processor. As such, the processor(s) may write to and read from the memory, and perform other common functions associated with managing semiconductor memory. Processing circuit(s) of the computing device 160 may be integrated circuits with power, input, and output pins capable of performing logic functions. In various implementations, the processor is a targeted device controller, a microprocessor configured to control one or components of the surgical system 100 and/or a combination thereof. The computing device 160 can be a part of the console 110, the fluidics module 200, and/or the optical pressure sensor 250.

The computing device 160 can generate and transmit control signals to the light source 252, the motors 210 of the fluidics module 200 that control irrigation and aspiration using the cassette 300, and/or other powered components of the surgical system 100. The computing device 160 can also receive signals representative of the light received at the sensor 254. The computing device 160 can process the signals received from the sensor 254 to determine distances between the optical pressure sensor 250 and components, such as the body 302, diaphragms 310, 320, and/or mounts 350, of the cassette 300. Based on the measured distances, the computing device 160 can further determine the vacuum pressure and/or fluid pressure associated with the irrigation fluid and/or aspiration fluid within the cassette 300. Exemplary methods of determining distance and pressure using an optical pressure sensor are described in U.S. Application No. 10/879,789, filed June 29, 2004, the entirety of which is hereby incorporated by reference. For example, the computing device 160 mathematically correlates a distance value (e.g., the displacement of the diaphragms 310, 320) to a pressure value. In some embodiments, the memory of the computing device 160 can store a look up table listing distance values and corresponding pressure values. The computing device 160 identifies the corresponding pressure value in the look up table once the distance value is determined.

During use, the nature of moveable parts introduces some level of variability in distances between the parts. For example, various components of the fluidics module 200 (e.g., the motors 210, the valve controls 220, the attachment members 230, the rollers 260) and the cassette 300 (e.g., the pump 330, the valves 340, the tubes 360) can introduce movement of the cassette 300 relative to the fluidics module 200. Movement the fluidics module 200 and/or the cassette 300 changes the distance between the optical pressure sensor 250 and the diaphragms 310, 320. Accordingly, the accuracy of the pressure determination can be adversely impacted by unaccounted movement of the fluidics module 200 and the cassette 300. To minimize this potential for inaccurate pressure measurement, the present disclosure describes measuring the distance between the optical pressure sensor 250 and a reference location on the cassette 300, as well as the distance between the optical pressure sensor 250 and the diaphragms 310, 320. The reference location, such as the mounts 350, experiences the same movement as the cassette 300 and/or the fluidics module 200. Therefore, even as the whole cassette 300 moves relative to the fluidics module 200, the distance between the diaphragms 310, 320 and the reference location on the cassette 300 is unaffected. By subtracting the two distances to find the difference, the displacement of the diaphragms 310, 320 due solely to pressure within the cassette 300 can be isolated. The computing device 160 can more accurately determine the pressure associated with the irrigation fluid and/or the aspiration fluid within the cassette based on the displacement of the diaphragms.

As illustrated in FIG. 5A, the light source 252 of the optical pressure sensor 250 is configured to output a light beam towards the fluidics cassette 300. In some embodiments, the optical pressure sensor 250 includes a beam splitter 256 configured to split the single light beam output by the light source 252 into multiple beams, such as two beams 258, 259 shown in the illustrated embodiment. The beam splitter 256 can include any suitable combination of lenses, mirrors, filters, gratings, and/or other optical components.

Beams 258, 259 interact with different components of the fluidics cassette 300. For example, the diaphragm 310 reflects at least a portion of the beam 258. The sensor 254 can be positioned to receive the reflected portion of the beam 258. The sensor 254 generates and transmits a signal representative of the reflected portion of the beam 258 to the computing device 160. Based on the received signal from the sensor 254, the computing device determines a distance 292 between the sensor 254 and the diaphragm 310.

The mount 350 reflects at least a portion of the beam 259. The sensor 254 can be positioned to receive the reflected portion of the beam 259. The sensor 254 generates and transmits a signal representative of the reflected portion of the beam 259 to the computing device 160. Based on the received signal from the sensor 254, the computing device determines a distance 294 between the sensor 254 and the mount 350.

The computing device 160 can process the received signals from the sensor 254 to isolate the displacement of the diaphragm 310 due to pressure associated with the irrigation fluid 304 within the body 302 of the cassette 300. In that regard, the mount 350 can serve as a reference location. For example, the computing device 160 can subtract the distance 294 from the distance 292 to yield a distance 296. Displacement of the diaphragm 310 changes the distance 296. By subtracting the distance 294, the computing device 160 minimizes any influence of unintended movement of fluidics module 200 and/or the cassette 300. In some implementations, the computing device 160 is arranged to determine the pressure associated with the irrigation fluid 304 within the cassette 300 using the look up table with the calculated distance 296. While the discussion of FIG. 5A refers specifically to the irrigation diaphragm 310 within the cassette 300, it is understand the disclosure is equally applicable with respect to the aspiration diaphragm 320.

FIG. 5B illustrates features similar to those shown in FIG. 5A, except that FIG. 5B additionally includes an additional light source 253 and an additional sensor 255. In that regard, the light source 253 can transmit the beam 259 that is reflected by the mount 350. The reflected portion of the beam 259 can be received at the sensor 255. The light source 252 can transmit the beam 258 that is reflected by the diaphragm 310. The reflected portion of the beam 258 can be received at the sensor 254. In some embodiments, the wavelength of light associated with the beams 258 and 259 are different to allow the sensor to readily distinguish between the beams.

In various embodiments, the system 100 can include a single light source and a single sensor, a single light source and multiple sensors, multiple light sources and a single sensor, multiple light sources and multiple sensors. In that regard, it is understood that the computing device 160 can determine the pressure associated with both the irrigation path 205 and the aspiration path 207. For example, the irrigation pressure and/or the aspiration pressure can be monitored in an ad hoc or on demand manner, at regular or irregular intervals, simultaneously and/or at times offset from one another. The optical pressure sensor 250 can include one or more light sources and one or more sensors to measure irrigation pressure based on deflection of the irrigation diaphragm 310. For example, the one or more sensors associated with the irrigation path 205 can respectively receive first and second reflected portions of light from the irrigation diaphragm 310 (e.g., the central portion 312 and/or the peripheral portion 314) and the reference portion of the fluidics cassette 300 (e.g., the mount 350 and/or the peripheral portion 314). The optical pressure sensor 250 can additionally include one or more light sources and one or more sensors to measure aspiration pressure based on deflection of the aspiration diaphragm 320. For example, the one or more sensors associated with the aspiration path 207 can respectively receive third and fourth reflected portions of light from the aspiration diaphragm 320 (e.g., the central portion 312 and/or the peripheral portion 314) and a reference portion of the fluidics cassette 300 (e.g., the mount 350 and/or the peripheral portion 314). The computing device 160 can be in communication with the light source(s) and sensor(s) associated with both the irrigation path 205 and the aspiration path 207. In some embodiments, the same light source(s) and the same sensor(s) are used to determine pressures associated with both the irrigation path 205 and the aspiration path 207.

FIG. 5C illustrates features similar to those shown in FIG. 5A, except that reference location is the peripheral portion 314 of the diaphragm. In that regard, the central portion 312 of the diaphragm 310 reflects at least a portion of the beam 258. The peripheral portion 314 of the diaphragm 310 reflects at least a portion of the beam 259. As described above, the central portion 312 may be more susceptible to deflection than the peripheral portion 314. The computing device 160 can determine the displacement of the diaphragm 310 based on the different distances between the sensor 254, and the central portion 312 and the peripheral portion 314 of the diaphragm 310. The computing device 160 can further determine the corresponding pressure associated with the irrigation fluid 304 within the cassette 300 based on the displacement of the diaphragm 310.

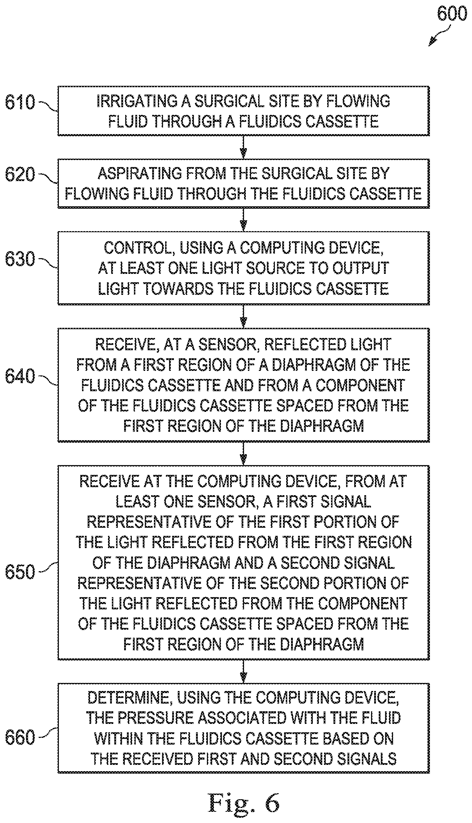

FIG. 6 illustrates a flowchart of an example ophthalmic surgical method 600. As illustrated, the method 600 includes a number of enumerated steps, but implementations of the method 600 may include additional steps before, after, and in between the enumerated steps. In some implementations, one or more of the enumerated steps may be omitted or performed in a different order. In some embodiments, the steps of method 600 can be executed by the computing device 160. In other embodiments, steps of the method 600 can be performed by other components of the system 100, as well as a surgeon or other medical professional.

The method 600 can be implemented during an ophthalmic surgical procedure, such as a cataract procedure, for example, or other suitable procedures. The surgeon may perform the surgical procedure using the components of the system 100. For example, the surgeon may perform surgical maneuvers using the handpiece 150 within the patient eye 160. The components of the system 100 can together facilitate irrigation and aspiration within the eye 160. The steps the method 600 can be implemented to monitor a pressure associated with irrigation fluid and/or aspiration fluid within the system 100.

At step 610, the method 600 includes irrigating a surgical site, such as the eye 160, by flowing fluid through the fluidics cassette 300. At step 620, the method 600 includes aspirating from the surgical site by flowing fluid through the fluidics cassette 300. The computing device 160 and/or a user, such as the surgeon or other medical professional, can coordinate delivery of irrigation fluid to the surgical site in step 610 and the removal of aspiration fluid from the surgical site in step 620 to maintain desired intraocular pressure during the surgical procedure. The step 610 can include the computing device 160 transmitting control signals to the irrigation bag 111, motors 200, and/or other powered components of the fluidics module 200 and/or the system 100 to facilitate delivery of irrigation fluid to the eye 160 using the fluidics cassette 300. Similarly, the step 620 can include the computing device 160 transmitting control signals to the motors 200 and/or other powered components of the fluidics module 200 and/or the system 100 to facilitate delivery of irrigation fluid to the eye 160 using the fluidics cassette 300.

At step 630, the method 600 includes controlling, using the computing device 160, at least one light source (e.g., the light source(s) 252, 253) to output light towards the fluidics cassette 300. The first portion of the light (e.g., beam 258) reflects from the central region 312 of the diaphragm 310, 320 of the fluidics cassette 300. A second portion of the light (e.g., the beam 259) is reflected from a component of the fluidics cassette 300 (e.g., the peripheral region 314, the mount 350) spaced from the central region 312 of the diaphragm 310, 320. In embodiments in which the beam 259 reflects from the mount 350, the beam 258 can be directed to any portion of the diaphragm 310, 320 (e.g., the central portion 312, the peripheral portion 314, etc.) The diaphragm 310, 320 is configured to deflect in response to a pressure associated with irrigation fluid and/or aspiration fluid within the fluidics cassette 300.

At step 640, the method 600 includes at least one sensor (e.g., the sensor(s) 254, 255) receiving reflected light from a first region (e.g., the central region 312 and/or the peripheral region 314) of the diaphragms 310, 320. Step 640 also includes the at least one sensor receiving reflected light from the reference component of the cassette 300 and/or the component of the cassette 300 spaced from the first region of the diaphragm (e.g., the peripheral region 314 and/or the mount 350). The at least one sensor is configured to generate respective electrical signals representative of the reflected light and transmit the signals to the computing device 160.

At step 650, the method 600 includes receiving first and second signals at the computing device 160, from the at least one sensor (e.g., the sensor(s) 254, 255). The first signal can be representative of the first portion of the light reflected from the central region 312 of the diaphragm 310, 320. The second signal can be representative of the second portion of the light reflected from the peripheral region 314 or the mount 350.

At step 660, the method 600 includes determining, using the computing device 160, the pressure (e.g., the fluid pressure and/or the vacuum pressure) associated with the irrigation fluid and/or aspiration fluid within the fluidics cassette 300 based on the received first and second signals. Determining the pressure of the irrigation fluid and/or aspiration fluid can include determining a first distance between the at least one sensor and the central region 312 of the diaphragm 310, 320 based on the received first signal. The method 600 can also include determining a second distance between the at least one sensor, and the peripheral region 314 or the mount 350, based on the received second signal. The method 600 can further include calculating a displacement of the diaphragm 310, 320 by subtracting the second distance from the first distance. The method 600 can also include correlating the displacement of the diaphragm 310, 320 to the pressure associated with the fluid within the fluidics cassette 300.

Once the pressure associated with the irrigation fluid and/or aspiration fluid is determined, the pressure can be increased, decreased, and/or kept the same in order to maintain proper intraocular pressure. For example, in response to the determined pressure(s), the computing device 160 can transmit control signals to the motors 210, the valve controls 220, the rollers 260, the pump 330, the valves 340, and/or other powered components of the system 100, to adjust the pressure associated with the irrigation fluid and/or aspiration fluid to maintain proper intraocular pressure. The user, such as the surgeon and/or other medical professional, can also monitor the measured pressure based on data displayed on the monitor 140. The user can adjust the aspiration and/or irrigation pressure as necessary.

Persons of ordinary skill in the art will appreciate that the implementations encompassed by the present disclosure are not limited to the particular exemplary implementations described above. In that regard, although illustrative implementations have been shown and described, a wide range of modification, change, combination, and substitution is contemplated in the foregoing disclosure.

## Claims

1. An ophthalmic surgical system, comprising:
at least one light source (252, 253) configured to output light towards a fluidics cassette (300) in a manner that a first portion (258) of the light reflects from a deflectable diaphragm (310) of the fluidics cassette, and in a manner that a second portion (259) of the light reflects from a reference portion (350, 314) of the fluidics cassette (300), wherein the diaphragm (310) is configured to deflect relative to the reference portion in response to a pressure associated with a first fluid (304) within the fluidics cassette;
at least one sensor (254, 255) configured to receive the first portion of the light reflected from the diaphragm (310) and the second portion of the light reflected from the reference portion (314, 350); and
a computing device (160) in communication with the at least one sensor, the computing device configured to determine the pressure associated with the first fluid within the fluidics cassette based on the received first and second portions of the light, and
**characterized in that**:
the at least one light source (252, 253) is configured to output light towards the fluidics cassette such that a third portion of the light reflects from a second diaphragm (320) of the fluidics cassette and a fourth portion of the light reflects from a second reference portion (314, 350) of the fluidics cassette, wherein the second diaphragm (320) is configured to be deflected in response to a pressure associated with a second fluid within the fluidics cassette (300);
the at least one sensor (254, 255) further configured to receive the third portion of the light reflected from the second diaphragm and the fourth portion of the light reflected from the second reference portion; and
the computing device (160) is configured to determine the pressure associated with the second fluid within the fluidics cassette based on the received third and fourth portions of the light.

2. The system of claim 1, further comprising:
a beam splitter (256) configured to direct the first portion of the light towards the diaphragm of the fluidics cassette and the second portion of the light towards the reference portion.

3. The system of claim 1, wherein the at least one sensor comprises:
a first sensor (254) configured to receive the first portion of the light reflected from the diaphragm; and
a second sensor (255) configured to receive the second portion of the light reflected from the reference portion.

4. The system of claim 1, wherein the at least one light source comprises:
a first light source (252) configured to output the first portion of the light; and
a second light source (253) configured to output the second portion of the light.

5. The system of claim 4, wherein the at least one sensor comprises:
a first sensor (254) configured to receive the first portion of the light reflected from the diaphragm; and
a second sensor (255) configured to receive the second portion of the light reflected from the reference portion.

6. The system of claim 1, wherein:
the pressure associated with the first fluid within the fluidics cassette (300) is representative of an irrigation pressure; and
the pressure associated with the second fluid within the fluidics cassette (300) is representative of an aspiration pressure.

7. The system of claim 1, wherein the at least one light source (252, 253) comprises a laser source or a laser diode.

8. The system of claim 1, further comprising:
a surgical console housing the at least one light source (252, 253), the at least one sensor (254, 255), and the computing device (160).

9. The system of claim 8, further comprising:
the fluidics cassette (300).

10. The system of claim 1, wherein the computing device (160) is configured to determine the pressure associated with the fluid within the fluidics cassette (300) by:
determining a first distance between the at least one sensor and the diaphragm based on the received first portion of the light reflected from the diaphragm; and
determining a second distance between the at least one sensor and the reference portion based on the received second portion of the light reflected from the reference portion.

11. The system of claim 10, wherein the computing device (160) is configured to determine the pressure associated with the fluid within the fluidics cassette (300) by: calculating a displacement of the diaphragm by subtracting the second distance from the first distance.

12. The system of claim 11, wherein the computing device (160) is configured to determine the pressure associated with the fluid within the fluidics cassette (300) by:
correlating the displacement of the diaphragm to the pressure associated with the fluid within the fluidics cassette.

13. A method of determining a pressure within an ophthalmic surgical system, the method comprising:
controlling (630), using a computing device (160), at least one light source (252, 253) to output light towards a fluidics cassette
such that a first portion of the light reflects from a region of a diaphragm (310) of the fluidics cassette and such that a second portion of the light reflects from a component of the fluidics cassette spaced from the region of the diaphragm, wherein the diaphragm (310) is configured to be deflected in response to a pressure associated with a first fluid within the fluidics cassette, and
such that a third portion of the light reflects from a second diaphragm (320) of the fluidics cassette and such that a fourth portion of the light reflects from a second reference portion of the fluidics cassette, wherein the second diaphragm (320) is configured to be deflected in response to a pressure associated with a second fluid within the fluidics cassette;
receiving (640) at the computing device, from at least one sensor,
a first signal representative of the first portion of the light reflected from the region of the diaphragm (310) and a second signal representative of the second portion (314, 350) of the light reflected from the component of the fluidics cassette spaced from the region of the diaphragm (310), and
a third signal representative of the third portion of the light reflected from the second diaphragm and a fourth signal representative of the fourth portion of the light reflected from the second reference portion;
determining (660), using the computing device,
the pressure associated with the first fluid within the fluidics cassette based on the received first and second signals, and
the pressure associated with the second fluid within the fluidics cassette based on the received third and fourth signals.

14. The method of claim 13, wherein determining the pressure associated with the fluid within the fluidics cassette comprises determining a first distance between the at least one sensor and the region of the diaphragm based on the received first signal; and
determining a second distance between the at least one sensor and the component of the fluidics cassette spaced from the region of the diaphragm based on the received second signal.

15. The method of claim 14, wherein determining the pressure associated with the fluid within the fluidics cassette comprises one of the following:
(i) calculating a displacement of the diaphragm by subtracting the second distance from the first distance;
(ii) calculating a displacement of the diaphragm by subtracting the second distance from the first distance and correlating the displacement of the diaphragm to the pressure associated with the fluid within the fluidics cassette.

## Patentansprüche

1. Augenchirurgisches System, das Folgendes umfasst:
mindestens eine Lichtquelle (252, 253), die konfiguriert ist, in Richtung einer Fluidik-Kassette (300) Licht auf eine Weise auszugeben, dass ein erster Anteil (258) des Lichts von einer auslenkbaren Membran (310) der Fluidik-Kassette reflektiert wird ein zweiter Anteil (259) des Lichts von einem Referenzabschnitt (350, 314) der Fluidik-Kassette (300) reflektiert wird, wobei die Membran (310) konfiguriert ist, als Antwort auf einen Druck, der einem ersten Fluid (304) in der Fluidik-Kassette zugeordnet ist, in Bezug auf den Referenzabschnitt ausgelenkt zu werden;
mindestens einen Sensor (254, 255), der konfiguriert ist, den ersten Anteil des Lichts, der von der Membran (310) reflektiert wird, und den zweiten Anteil des Lichts, der vom Referenzabschnitt (314, 350) reflektiert wird, zu empfangen; und
eine Rechenvorrichtung (160) in Verbindung mit dem mindestens einen Sensor, wobei die Rechenvorrichtung konfiguriert ist, auf der Grundlage des empfangenen ersten und zweiten Anteils des Lichts den Druck zu bestimmen, der dem ersten Fluid in der Fluidik-Kassette zugeordnet ist, und
**dadurch gekennzeichnet, dass**:
die mindestens eine Lichtquelle (252, 253) konfiguriert ist, Licht in Richtung der Fluidik-Kassette auszugeben, derart, dass ein dritter Anteil des Lichts von einer zweiten Membran (320) der Fluidik-Kassette reflektiert wird und ein vierter Anteil des Lichts von einem zweiten Referenzabschnitt (314, 350) der Fluidik-Kassette reflektiert wird, wobei die zweite Membran (320) konfiguriert ist, als Antwort auf einen Druck, der einem zweiten Fluid in der Fluidik-Kassette (300) zugeordnet ist, ausgelenkt zu werden;
der mindestens eine Sensor (254, 255) ferner konfiguriert ist, den dritten Anteils des Lichts, der von der zweiten Membran reflektiert wird, und den vierten Anteil des Lichts, der vom zweiten Referenzabschnitt reflektiert wird, zu empfangen; und
die Rechenvorrichtung (160) konfiguriert ist, auf der Grundlage des empfangenen dritten und vierten Anteils des Lichts den Druck zu bestimmen, der dem zweiten Fluid in der Fluidik-Kassette zugeordnet ist.

2. System nach Anspruch 1, das ferner Folgendes umfasst:
einen Strahlteiler (256), der konfiguriert ist, den ersten Anteil des Lichts in Richtung der Membran der Fluidik-Kassette und den zweiten Anteil des Lichts in Richtung des Referenzabschnitts zu lenken.

3. System nach Anspruch 1, wobei der mindestens eine Sensor Folgendes umfasst:
einen ersten Sensor (254), der konfiguriert ist, den ersten Anteil des Lichts, der von der Membran reflektiert wird, zu empfangen; und
einen zweiten Sensor (255), der konfiguriert ist, den zweiten Anteil des Lichts, der vom Referenzabschnitt reflektiert wird, zu empfangen.

4. System nach Anspruch 1, wobei die mindestens eine Lichtquelle Folgendes umfasst:
eine erste Lichtquelle (252), die konfiguriert ist, den ersten Anteil des Lichts auszugeben; und
eine zweite Lichtquelle (253), die konfiguriert ist, den zweiten Anteil des Lichts auszugeben.

5. System nach Anspruch 4, wobei der mindestens eine Sensor Folgendes umfasst:
einen ersten Sensor (254), der konfiguriert ist, den ersten Anteil des Lichts, der von der Membran reflektiert wird, zu empfangen; und
einen zweiten Sensor (255), der konfiguriert ist, den zweiten Anteil des Lichts, der vom Referenzabschnitt reflektiert wird, zu empfangen.

6. System nach Anspruch 1, wobei:
der Druck, der dem ersten Fluid in der Fluidik-Kassette (300) zugeordnet ist, einen Spülungsdruck darstellt; und
der Druck, der dem zweiten Fluid in der Fluidik-Kassette (300) zugeordnet ist, einen Absaugdruck darstellt.

7. System nach Anspruch 1, wobei die mindestens eine Lichtquelle (252, 253) eine Laserquelle oder eine Laserdiode umfasst.

8. System nach Anspruch 1, das ferner Folgendes umfasst:
eine Operationskonsole, die die mindestens eine Lichtquelle (252, 253), den mindestens einen Sensor (254, 255) und die Rechenvorrichtung (160) aufnimmt.

9. System nach Anspruch 8, das ferner Folgendes umfasst:
die Fluidik-Kassette (300).

10. System nach Anspruch 1, wobei die Rechenvorrichtung (160) konfiguriert ist, den Druck, der dem Fluid in der Fluidik-Kassette (300) zugeordnet ist, zu bestimmen durch:
Bestimmen eines ersten Abstands zwischen dem mindestens einen Sensor und der Membran auf der Grundlage des empfangenen ersten Anteils des Lichts, der von der Membran reflektiert wird; und
Bestimmen eines zweiten Abstands zwischen dem mindestens einen Sensor und dem Referenzabschnitt auf der Grundlage des empfangenen zweiten Anteils des Lichts, der vom Referenzabschnitt reflektiert wird.

11. System nach Anspruch 10, wobei die Rechenvorrichtung (160) konfiguriert ist, den Druck, der dem Fluid in der Fluidik-Kassette (300) zugeordnet ist, zu bestimmen durch:
Berechnen einer Verlagerung der Membran durch Subtrahieren des zweiten Abstands vom ersten Abstand.

12. System nach Anspruch 11, wobei die Rechenvorrichtung (160) konfiguriert ist, den Druck, der dem Fluid in der Fluidik-Kassette (300) zugeordnet ist, zu bestimmen durch:
Korrelieren der Verlagerung der Membran mit dem Druck, der dem Fluid in der Fluidik-Kassette zugeordnet ist.

13. Verfahren zum Bestimmen eines Drucks in einem augenchirurgischen System, wobei das Verfahren Folgendes umfasst:
Steuern (630) mindestens einer Lichtquelle (252, 253) unter Verwendung einer Rechenvorrichtung (160), derart, dass Licht in Richtung einer Fluidik-Kassette ausgegeben wird,
derart, dass ein erster Anteil des Lichts von einem Bereich einer Membran (310) der Fluidik-Kassette reflektiert wird, und derart, dass ein zweiter Anteil des Lichts von einer Komponente der Fluidik-Kassette reflektiert wird, die von dem Bereich der Membran beabstandet ist, wobei die Membran (310) konfiguriert ist, als Antwort auf einen Druck, der einem ersten Fluid in der Fluidik-Kassette zugeordnet ist, ausgelenkt zu werden, und
derart, dass ein dritter Anteil des Lichts von einer zweiten Membran (320) der Fluidik-Kassette reflektiert wird, und derart, dass ein vierter Anteil des Lichts von einem zweiten Referenzabschnitt der Fluidik-Kassette reflektiert wird, wobei die zweite Membran (320) konfiguriert ist, als Antwort auf einen Druck, der einem zweiten Fluid in der Fluidik-Kassette zugeordnet ist, ausgelenkt zu werden;
Empfangen (640) von dem mindestens einen Sensor an der Rechenvorrichtung
eines ersten Signals, das den ersten Anteil des Lichts darstellt, der von dem Bereich der Membran (310) reflektiert wird, und eines zweiten Signals, das den zweiten Anteil (314, 350) des Lichts darstellt, der von der Komponente der Fluidik-Kassette reflektiert wird, die von dem Bereich der Membran (310) beabstandet ist, und
eines dritten Signals, das den dritten Anteil des Lichts darstellt, der von der zweiten Membran reflektiert wird, und eines vierten Signals, das den vierten Anteil des Lichts darstellt, der vom zweiten Referenzabschnitt reflektiert wird;
Bestimmen (660) unter Verwendung der Rechenvorrichtung
des Drucks, der dem ersten Fluid in der Fluidik-Kassette zugeordnet ist, auf der Grundlage des empfangenen ersten und zweiten Signals, und
des Drucks, der dem zweiten Fluid in der Fluidik-Kassette zugeordnet ist, auf der Grundlage des empfangenen dritten und vierten Signals.

14. Verfahren nach Anspruch 13, wobei das Bestimmen des Drucks, der dem Fluid in der Fluidik-Kassette zugeordnet ist, Folgendes umfasst:
Bestimmen eines ersten Abstands zwischen dem mindestens einen Sensor und dem Bereich der Membran auf der Grundlage des empfangenen ersten Signals; und
Bestimmen eines zweiten Abstands zwischen dem mindestens einen Sensor und der Komponente der Fluidik-Kassette, die von dem Bereich der Membran beabstandet ist, auf der Grundlage des empfangenen zweiten Signals.

15. Verfahren nach Anspruch 14, wobei das Bestimmen des Drucks, der dem Fluid in der Fluidik-Kassette zugeordnet ist, Folgendes umfasst:
entweder
(i) Berechnen einer Verlagerung der Membran durch Subtrahieren des zweiten Abstands vom ersten Abstand;
oder
(ii) Berechnen einer Verlagerung der Membran durch Subtrahieren des zweiten Abstands vom ersten Abstand und Korrelieren der Verlagerung der Membran mit dem Druck, der dem Fluid in der Fluidik-Kassette zugeordnet ist.

## Revendications

1. Système chirurgical ophtalmologique, comprenant :
au moins une source lumineuse (252, 253) configurée pour émettre de la lumière vers une cassette fluidique (300) de manière à ce qu'une première partie (258) de la lumière soit réfléchie par une membrane déformable (310) de la cassette fluidique, et de manière à ce qu'une deuxième partie (259) de la lumière soit réfléchie par une partie de référence (350, 314) de la cassette fluidique (300), la membrane (310) étant configurée pour se déformer par rapport à la partie de référence en réponse à une pression associée à un premier fluide (304) au sein de la cassette fluidique ;
au moins un capteur (254, 255) configuré pour recevoir la première partie de la lumière réfléchie par la membrane (310) et la deuxième partie de la lumière réfléchie par la partie de référence (314, 350) ; et
un dispositif informatique (160) en communication avec l'au moins un capteur, le dispositif informatique étant configuré pour déterminer la pression associée au premier fluide au sein de la cassette fluidique en fonction des première et deuxième parties reçues de la lumière, et
**caractérisé en ce que** :
l'au moins une source lumineuse (252, 253) est configurée pour émettre de la lumière vers la cassette fluidique de sorte qu'une troisième partie de la lumière soit réfléchie par une deuxième membrane (320) de la cassette fluidique et qu'une quatrième partie de la lumière soit réfléchie par une deuxième partie de référence (314, 350) de la cassette fluidique, la deuxième membrane (320) étant configurée pour se déformer en réponse à une pression associée à un deuxième fluide au sein de la cassette fluidique (300) ;
l'au moins un capteur (254, 255) est configuré en outre pour recevoir la troisième partie de la lumière réfléchie par la deuxième membrane et la quatrième partie de la lumière réfléchie par la deuxième partie de référence ; et
le dispositif informatique (160) est configuré pour déterminer la pression associée au deuxième fluide au sein de la cassette fluidique en fonction des troisième et quatrième parties reçues de la lumière.

2. Système selon la revendication 1, comprenant en outre :
un séparateur de faisceau (256) configuré pour diriger la première partie de la lumière vers la membrane de la cassette fluidique et la deuxième partie de la lumière vers la partie de référence.

3. Système selon la revendication 1, dans lequel l'au moins un capteur comprend :
un premier capteur (254) configuré pour recevoir la première partie de la lumière réfléchie par la membrane ; et
un deuxième capteur (255) configuré pour recevoir la deuxième partie de la lumière réfléchie par la partie de référence.

4. Système selon la revendication 1, dans lequel l'au moins une source lumineuse comprend :
une première source lumineuse (252) configurée pour émettre la première partie de la lumière ; et
une deuxième source lumineuse (253) configurée pour émettre la deuxième partie de la lumière.

5. Système selon la revendication 4, dans lequel l'au moins un capteur comprend :
un premier capteur (254) configuré pour recevoir la première partie de la lumière réfléchie par la membrane ; et
un deuxième capteur (255) configuré pour recevoir la deuxième partie de la lumière réfléchie par la partie de référence.

6. Système selon la revendication 1, dans lequel :
la pression associée au premier fluide au sein de la cassette fluidique (300) est représentative d'une pression d'irrigation ; et
la pression associée au deuxième fluide au sein de la cassette fluidique (300) est représentative d'une pression d'aspiration.

7. Système selon la revendication 1, dans lequel l'au moins une source lumineuse (252, 253) comprend une source laser ou une diode laser.

8. Système selon la revendication 1, comprenant en outre :
une console chirurgicale abritant l'au moins une source lumineuse (252, 253), l'au moins un capteur (254, 255) et le dispositif informatique (160).

9. Système selon la revendication 8, comprenant en outre :
la cassette fluidique (300).

10. Système selon la revendication 1, dans lequel le dispositif informatique (160) est configuré pour déterminer la pression associée au fluide au sein de la cassette fluidique (300) en :
déterminant une première distance entre l'au moins un capteur et la membrane en fonction de la première partie reçue de la lumière réfléchie par la membrane ; et
déterminant une deuxième distance entre l'au moins un capteur et la partie de référence en fonction de la deuxième partie reçue de la lumière réfléchie par la partie de référence.

11. Système selon la revendication 10, dans lequel le dispositif informatique (160) est configuré pour déterminer la pression associée au fluide au sein de la cassette fluidique (300) en :
calculant un déplacement de la membrane en soustrayant la deuxième distance à la première distance.

12. Système selon la revendication 11, dans lequel le dispositif informatique (160) est configuré pour déterminer la pression associée au fluide au sein de la cassette fluidique (300) en :
corrélant le déplacement de la membrane à la pression associée au fluide au sein de la cassette fluidique.

13. Procédé de détermination d'un pression au sein d'un système chirurgical ophtalmologique, le procédé comprenant :
la commande (630), au moyen d'un dispositif informatique (160), à au moins une source lumineuse (252, 253) d'émettre de la lumière vers une cassette fluidique
de manière à ce qu'une première partie de la lumière soit réfléchie par une région d'une membrane (310) de la cassette fluidique, et de manière à ce qu'une deuxième partie de la lumière soit réfléchie par un composant de la cassette fluidique espacé de la région de la membrane, la membrane (310) étant configurée pour se déformer en réponse à une pression associée à un premier fluide au sein de la cassette fluidique, et
de manière à ce qu'une troisième partie de la lumière soit réfléchie par une deuxième membrane (320) de la cassette fluidique et qu'une quatrième partie de la lumière soit réfléchie par une deuxième partie de référence de la cassette fluidique, la deuxième membrane (320) étant configurée pour se déformer en réponse à une pression associée à un deuxième fluide au sein de la cassette fluidique ;
la réception (640), au niveau du dispositif informatique, depuis au moins un capteur,
d'un premier signal représentatif de la première partie de la lumière réfléchie par la région de la membrane (310) et d'un deuxième signal représentatif de la deuxième partie (314, 350) de la lumière réfléchie par le composant de la cassette fluidique espacé de la région de la membrane (310), et
d'un troisième signal représentatif de la troisième partie de la lumière réfléchie par la deuxième membrane et d'un quatrième signal représentatif de la quatrième partie de la lumière réfléchie par la deuxième partie de référence ;
la détermination (660), au moyen du dispositif informatique,
de la pression associée au premier fluide au sein de la cassette fluidique en fonction des premier et deuxième signaux reçus, et
de la pression associée au deuxième fluide au sein de la cassette fluidique en fonction des troisième et quatrième signaux reçus.

14. Procédé selon la revendication 13, dans lequel la détermination de la pression associée au fluide au sein de la cassette fluidique comprend la détermination d'une première distance entre l'au moins un capteur et la région de la membrane en fonction du premier signal reçu ; et
la détermination d'une deuxième distance entre l'au moins un capteur et le composant de la cassette fluidique espacé de la région de la membrane en fonction du deuxième signal reçu.

15. Procédé selon la revendication 14, dans lequel la détermination de la pression associée au fluide au sein de la cassette fluidique comprend :
soit (i) le calcul d'un déplacement de la membrane par soustraction de la deuxième distance à la première distance ;
soit (ii) le calcul d'un déplacement de la membrane par soustraction de la deuxième distance à la première distance et la corrélation du déplacement de la membrane à la pression associée au fluide au sein de la cassette fluidique.
